# EUROPÄISCHE PATENTSCHRIFT

(11) **EP 3 344 499 B1**
(45) Veröffentlichungstag und Bekanntmachung des Hinweises auf die Patenterteilung: **10.07.2019**
(21) Anmeldenummer: 16760663.1
(22) Anmeldetag: 16.08.2016
(51) Int. Cl.: B60R 22/12, A61B 5/00

(54) **SICHERHEITSGURTANORDNUNGEN FÜR EIN KRAFTFAHRZEUG UND VERFAHREN ZUM HERSTELLEN DERARTIGER SICHERHEITSGURTANORDNUNGEN**
SAFETY BELT APPARATUS FOR A MOTOR VEHICLE AND METHOD FOR PRODUCING SUCH SAFETY BELT APPARATUS
AGENCEMENTS DE CEINTURE DE SÉCURITÉ DESTINÉS À UN VÉHICULE À MOTEUR ET PROCÉDÉ DE RÉALISATION DE TELS AGENCEMENTS DE CEINTURE DE SÉCURITÉ

(30) Priorität: 04.09.2015 DE 102015217028
(43) Veröffentlichungstag der Anmeldung: 11.07.2018
(73) Patentinhaber: Joyson Safety Systems Germany GmbH, 63743 Aschaffenburg (DE)
(72) Erfinder: PAUSCH, Tobias, 10435 Berlin (DE)
(74) Vertreter: Maikowski & Ninnemann Patentanwälte Partnerschaft mbB
(86) Internationale Anmeldenummer: PCT/EP2016/069412
(87) Internationale Veröffentlichungsnummer: WO 2017/036788

(56) Entgegenhaltungen:
- EP-A1- 2 174 590
- WO-A1-2014/102459
- DE-A1- 10 327 753
- JP-A- 2001 260 698
- US-A1- 2007 182 534

## Beschreibung

Die Erfindung betrifft eine Sicherheitsgurtanordnung für ein Kraftfahrzeug gemäß dem Oberbegriff des Anspruchs 1 sowie ein Verfahren zum Herstellen einer Sicherheitsgurtanordnung gemäß Anspruch 6.

Aus dem Stand der Technik ist bekannt, Sensoren an einem Sicherheitsgurt eines Kraftfahrzeuges anzubringen, wobei die Sensoren zum Beispiel zur Überwachung von Vitalfunktionen eines Gurtbenutzers dienen. Eine derartige Sicherheitsgurtanordnung ist beispielsweise in der DE 103 27 753 A1 beschrieben, wobei sich dort die Sensoren als Erhebungen auf einer dem Gurtbenutzer abgewandten Seite des Sicherheitsgurtes befinden. Diese Art der Anordnung der Sensoren kann jedoch die Qualität der Sensorsignale beeinträchtigen.

Die JP 2001 260 698 A beschreibt eine Sicherheitsgurtanordnung für ein Kraftfahrzeug, bei dem ein Sensor an einer dem Gurtbenutzer zugewandten Seite eines Sicherheitsgurtes angeordnet ist.

Das der Erfindung zugrunde liegende Problem besteht darin, eine möglichst einfach herstellbare Sicherheitsgurtanordnung zu schaffen, die Sensorsignale möglichst guter Qualität ermöglicht.

Dieses Problem wird durch die Sicherheitsgurtanordnung mit den Merkmalen des Anspruchs 1 sowie durch das Herstellungsverfahren mit den Merkmalen gemäß Anspruch 6 gelöst.

Beispielsweise umfasst eine Sicherheitsgurtanordnung für ein Kraftfahrzeug einen Sicherheitsgurt;
- mindestens einen Sensor, wobei
- der Sensor in einer Einbuchtung einer einem Benutzer des Sicherheitsgurtes zuzuwendenden Innenseite des Sicherheitsgurtes angeordnet ist.

Durch die Anordnung des Sensors oder der mehreren Sensoren auf einer Innenseite des Sicherheitsgurtes wird ein möglichst guter Kontakt und/oder möglichst geringer Abstand zwischen dem Sensor und dem Benutzer des Sicherheitsgurtes (dem Fahrzeuginsassen) realisiert, um Sensorsignale möglichst guter Qualität zu erhalten. Die Anordnung des Sensors oder der mehreren Sensoren erfolgt insbesondere derart, dass deren Sensierrichtung zum Fahrzeuginsassen hin gerichtet ist. Gleichzeitig wird durch die Anordnung des Sensors in einer Einbuchtung des Sicherheitsgurtes mechanischen Problemen bei der Benutzung des Gurtes entgegengewirkt. Beispielsweise wird vermieden, dass der Sensor an einem Gurtumlenkelement der Sicherheitsgurtanordnung hängenbleibt und das Ab- oder Aufwickeln des Sicherheitsgurtes blockiert oder gebremst wird. Modifikationen (etwa das Einbringen von Aussparungen) von Gurtführungen und/oder Gurtumlenkungselementen der Sicherheitsgurtanordnung sind daher nicht erforderlich.

Dass die (dauerhafte) Einbuchtung in der Innenseite des Sicherheitsgurtes ausgebildet ist, bedeutet, dass sie so ausgeformt ist, dass der Sensor gleichzeitig in der Einbuchtung und an der Innenseite des Sicherheitsgurtes angeordnet sein kann. Die Einbuchtung ist also eine von der Innenseite des Sicherheitsgurtes ausgehende Delle und macht sich entsprechend auf der anderen Seite (der Außenseite) des Sicherheitsgurtes als Ausbuchtung bemerkbar. Die Einbuchtung wird durch Deformation des Sicherheitsgurtes, d.h. durch Verformen des vorhandenen Materials, aus dem der Sicherheitsgurt gebildet ist, erzeugt. Es wird also zur Ausbildung der Einbuchtung kein zusätzliches Element an dem Sicherheitsgurt angeordnet.

Es wird darauf hingewiesen, dass unter einem "Sensor" nicht zwingend nur ein sensierendes Element verstanden werden soll, sondern z.B. auch eine Einheit (ein Modul), die neben dem sensierenden Element (etwa ein Halbleiterbauelement) weitere (insbesondere elektronische) Komponenten umfasst. Beispielsweise weist der Sensor ein sensierendes Bauelement auf, das auf einer Leiterplatte angeordnet ist. Denkbar ist auch, dass mehrere sensierende Elemente vorhanden sind, die zum Beispiel auf einer gemeinsamen Leiterplatte angeordnet sind.

Denkbar ist auch, dass der Sensor Bestandteil einer in der Einbuchtung der Sicherheitsanordnung angeordneten Sensoreinheit (Sensorknoten) ist, die mehrere Sensoren umfasst. Möglich ist hierbei, dass die Sensoren jeweils ein sensierendes Bauelement umfassen, wobei die sensierenden Bauelemente auf einer gemeinsamen Leiterplatte angeordnet sind. Beispiele des prinzipiellen Aufbaus des Sensors oder der Sensoreinheit, deren Funktion sowie deren Anordnung am Sicherheitsgurt sind in der deutschen Patentanmeldung 10 2014 211 501 vom 16.06.2014 beschrieben, auf die insofern ausdrücklich Bezug genommen wird. Beispielsweise ist der Sensor zum Ermitteln von Vitalparametern des Gurtbenutzers (z.B. seiner Herz- und/oder Atemaktivität) und/oder zum Ermitteln von Umgebungsgrößen (z.B. einer Umgebungstemperatur) ausgebildet. Denkbar ist, dass der Sensor hierfür zur Ermittlung von elektrischen Größen oder als akustischer Wandler (insbesondere als Mikrofon) ausgestaltet ist. Die Erfindung ist jedoch selbstverständlich nicht auf eine bestimmte Ausgestaltung des Sensors oder der Sensoren beschränkt.

Beispielsweise kann der am Sicherheitsgurt angeordnete Sensor (bzw. die dort angeordneten mehreren Sensoren) auch dazu dienen, die Position des Fahrzeuginsassen relativ zu dem Fahrzeuginnenraum zu ermitteln; insbesondere, um festzustellen, ob sich der Fahrzeuginsassen in einer anderen als der normalen Sitzposition befindet (d.h. eine "out-of-position"-Situation vorliegt). Abhängig von der ermittelten Sitzposition kann z.B. eine Anpassung (insbesondere eine Leistungsanpassung) eines Rückhaltesystems (z.B. eines Airbagsystems) des Fahrzeugs erfolgen. So könnte etwa abhängig von der ermittelten Position des Fahrzeuginsassen ein Entlüftungsmechanismus eines Airbags aktiviert werden. Als Sensor zur Positionsermittlung könnte z.B. ein (insbesondere dreiachsiger) Beschleunigungssensor verwendet werden, wobei anhand der Lage des Sensors die Insassenposition ermittelbar ist.

Bei dem Sicherheitsgurt handelt es sich z.B. um einen konventionellen Sicherheitsgurt, der insbesondere aus einem textilen Material gefertigt ist.

Die Einbuchtung besitzt insbesondere eine derartige Tiefe, dass der Sensor, z.B. auch die erwähnte Sensoreinheit, nicht oder nur in geringem Maße (z.B. um maximal 10 % der Tiefe der Einbuchtung) aus der Einbuchtung herausragt. Dadurch wird bei einer Anordnung des Sensors auf der Innenseite des Sicherheitsgurtes ein möglichst angenehmes Tragegefühl ermöglicht, so dass der Gurt z.B. auch dann nicht stört, wenn der Gurtbenutzer leichtere Kleindung trägt. Der Sensor ist hierbei dennoch insbesondere so angeordnet, dass der Abstand zum Fahrzeuginsassen nicht zu groß wird, um eine möglichst gute Qualität der Sensorsignale sicherzustellen. Beispielsweise schließt eine (dem Boden der Einbuchtung abgewandte) Oberseite des Sensors mit der Einbuchtung ab, d.h. die Oberseite liegt zumindest näherungsweise in einer Ebene mit dem die Einbuchtung umgebenden Abschnitt des Sicherheitsgurtes (bezogen auf einen flach ausgebreiteten Zustand dieses Bereiches des Sicherheitsgurtes). Denkbar ist allerdings auch, dass der Sensor oder die Sensoreinheit weiter aus der Einbuchtung herausragt, um ein besseres Anliegen des Sensors oder der Sensoreinheit an dem Körper des Fahrzeuginsassen zu ermöglichen.

Der Sensor ist insbesondere in der Einbuchtung mit dem Sicherheitsgurt verbunden. Die Verbindung zwischen dem Sensor und dem Sicherheitsgurt in der Einbuchtung erfolgt zum Beispiel stoffschlüssig (etwa durch Verkleben oder Verschweißen), insbesondere wird der Sensor zumindest mit einem Boden der Einbuchtung verbunden.

Gemäß einer anderen Weiterbildung der Sicherheitsgurtanordnung ist der Sensor zumindest teilweise von einem Schutzmaterial umgeben. Bei dem Schutzmaterial handelt es sich insbesondere um ein Polymer, das den Sensor vor äußeren Einflüssen (zum Beispiel Feuchtigkeit und/oder Verunreinigungen) schützen soll. Denkbar ist, dass das Schutzmaterial ein Vergussmaterial ist, in das der Sensor zumindest teilweise eingegossen ist.

Das Schutzmaterial kann so ausgebildet sein, dass es die Einbuchtung zumindest näherungsweise vollständig ausfüllt, d.h. das Schutzmaterial füllt das vom Sensor nicht eingenommene Restvolumen der Einbuchtung zumindest näherungsweise komplett aus.

Die Erfindung betrifft eine Sicherheitsgurtanordnung für ein Kraftfahrzeug, mit
- einem Sicherheitsgurt;
- mindestens einem Sensor, wobei
- der Sensor zumindest teilweise in einer durch Verdrängung von Material des Sicherheitsgurtes gebildeten Öffnung des Sicherheitsgurtes angeordnet ist.

Der Sensor ragt zumindest in die Öffnung hinein, wobei denkbar ist, dass ein Abschnitt des Sensors (z.B. eine Leiterplatte) oder ein anderes mit dem Sensor verbundenes Bauelement zumindest teilweise außerhalb der Öffnung angeordnet ist (z.B. an einer Seite des Sicherheitsgurtes anliegen). Auch hier erfolgt die Anordnung des Sensors insbesondere derart, dass deren Sensierrichtung zum Fahrzeuginsassen hin gerichtet ist. Bei der Öffnung handelt es sich z.B. um eine Durchgangsöffnung.

Der Sensor ist insbesondere wie oben beschrieben ausgebildet. Möglich ist natürlich auch, dass andere kompatible Ausgestaltungen der Sicherheitsgurtanordnung, die oben beschrieben wurden, in der Sicherheitsgurtanordnung gemäß der Erfindung realisiert sind.

Wie die oben beschriebene Einbuchtung wird die Öffnung zumindest nahezu ausschließlich durch Deformation eines Abschnittes des Sicherheitsgurtes, vorliegend durch (z.B. elastische) Materialverdrängung (Verschiebung von Material) erzeugt und nicht durch Zerstören (etwa Durchtrennen) des Materials des Sicherheitsgurtes. Dies stellt sicher, dass die Festigkeit des Sicherheitsgurtes möglichst wenig (insbesondere gar nicht) beeinträchtigt wird und insbesondere die gesetzlich geforderte Zugfestigkeit erhalten bleibt.

Der Sicherheitsgurt besteht aus einem gewebten Material, wobei die Öffnung durch Auseinanderdrücken von Materialfäden (z.B. von Kett- und/oder Schussfäden) des gewebten Materials gebildet ist. Möglich ist auch, dass ein vom Sensor nicht eingenommenes Volumen der Öffnung zumindest teilweise mit einem Schutzmaterial (z.B. dem oben erwähnten Vergussmaterial) versehen ist.

In einer anderen Ausgestaltung der Erfindung ist ein Halteelement vorhanden, das mit einem ersten Abschnitt an einer Seite des Sicherheitsgurtes anliegt und mit einem zweiten Abschnitt in die Öffnung hineinragt und mit dem Sensor verbunden ist. Möglich ist auch, dass der Sensor oder ein mit dem Sensor verbundenes Teil an der anderen Seite des Sicherheitsgurtes anliegt, so dass eine nietartige Befestigung des Sensors an dem Sicherheitsgurt erfolgt. Denkbar ist natürlich auch, dass der Sensor auf anderer Weise mit dem Sicherheitsgurt verbunden ist (s.o.).

Ein Verfahren zur Herstellung einer wie oben beschriebenen Sicherheitsgurtanordnung umfasst die Schritte:
- Bereitstellen eines Sicherheitsgurtes;
- Erzeugen einer Einbuchtung einer einem Benutzer des Sicherheitsgurtes zuzuwendenden Innenseite des Sicherheitsgurts; und
- Anordnen eines Sensors in der Einbuchtung.

Die Einbuchtung wird demnach so erzeugt, dass der Sensor an einer einem Benutzer des Sicherheitsgurtes zuzuwendenden Innenseite des Sicherheitsgurtes angeordnet werden kann. Beispielsweise bildet ein Boden und ein Randbereich der Einbuchtung einen Abschnitt der nach Anlegen des Sicherheitsgurtes dem Benutzer zugewandten Innenseite des Sicherheitsgurtes.

Gemäß einer Variante wird der Sensor nach seinem Anordnen in der Einbuchtung zumindest teilweise mit einem Schutzmaterial umgeben, wie oben bereits erwähnt. Das Schutzmaterial kann wie oben ebenfalls bereits erläutert in Form eines Vergussmaterials ausgebildet sein.

Gemäß einer alternativen Variante wird der Sensor (bzw. die oben erwähnte Sensoreinheit) bereits vor dem Anordnen in der Einbuchtung zumindest teilweise mit einem Schutzmaterial umgeben (verkapselt), wobei anschließend die aus dem Sensor und dem Schutzmaterial gebildete Einheit in der Einbuchtung angeordnet und dort dann zum Beispiel mit dem Sicherheitsgurt stoffschlüssig verbunden wird. Die Verkapselung des Sensors erfolgt insbesondere derart, dass die Abmessungen der Einheit aus dem Sensor und dem Schutzmaterial zumindest näherungsweise den Abmessungen der Einbuchtung entsprechen, so dass die Einheit nach ihrem Einsetzen in die Einbuchtung diese zumindest im Wesentlichen komplett ausfüllt.

Es wird darauf hingewiesen, dass die oben erläuterten Weiterbildungen der erfindungsgemäßen Sicherheitsgurtanordnung natürlich analog zur Weiterbildung des erfindungsgemäßen Verfahrens verwendet werden können.

Gemäß einer anderen Ausgestaltung des Verfahrens wird der Sicherheitsgurt zur Erzeugung der Einbuchtung zwischen zwei Werkzeugteilen positioniert, wobei das eine der Werkzeugteile eine mit der zu erzeugenden Einbuchtung korrespondierende Vertiefung aufweist, und wobei eine Relativbewegung der Werkzeugteile ausgeführt wird, derart, dass der Sicherheitsgurt in die Vertiefung des Werkzeugteils gedrückt wird. Insbesondere stellt das Werkzeugteil mit der Vertiefung eine Matrize und das andere Werkzeugteil einen Stempel dar, wobei der Stempel eine komplementär zur Vertiefung der Matrize ausgebildete Form aufweist.

Die Relativbewegung der beiden Werkzeugteile zueinander (aufeinander zu) wird insbesondere dadurch erzeugt, dass das Werkzeugteil mit der Vertiefung (die Matrize) in Ruhe bleibt und das andere Werkzeugteil (der Stempel) auf dieses Werkzeugteil zu bewegt wird und dabei den Sicherheitsgurt in die Vertiefung drückt. Denkbar ist allerdings auch, dass der Stempel in Ruhe bleibt und die Matrize auf den Stempel zu bewegt wird. Grundsätzlich ist natürlich auch möglich, dass beide Werkzeugteile bewegt werden.

Gemäß einer weiteren Variante erfolgt das Erzeugen der Einbuchtung in dem Sicherheitsgurt unter Erwärmung zumindest eines Teilabschnitts des Sicherheitsgurtes (insbesondere natürlich desjenigen Teilabschnitts, in dem die Einbuchtung entstehen soll). Das Erwärmen des Sicherheitsgurtes kann zum Beispiel dadurch erfolgen, dass zumindest eines der oben beschriebenen Werkzeugteile erwärmt und die Einbuchtung unter Verwendung der erwärmten Werkzeugteile hergestellt wird.

Die Erfindung betrifft auch ein Verfahren zur Herstellung einer Sicherheitsgurtanordnung, mit den Schritten:
- Bereitstellen eines Sicherheitsgurtes;
- Verdrängen von Material des Sicherheitsgurtes derart, dass eine Öffnung in dem Sicherheitsgurt entsteht; und
- Anordnen eines Sensors in der Öffnung.

Der Sicherheitsgurt besteht wie oben bereits erwähnt aus einem gewebten Material, wobei die Deformation eines Abschnittes des Sicherheitsgurtes, vorliegend das Verdrängen von Material des Sicherheitsgurtes durch Auseinanderdrücken von Materialfäden des gewebten Materials erfolgt. Das Auseinanderdrücken der Materialfäden erfolgt insbesondere mit Hilfe eines Werkzeuges (z.B. in Form eines Domes), wobei das Werkzeug einen konischen (insbesondere spitzen) Abschnitt aufweist, wobei das Werkzeug so bewegt wird, dass der konische Abschnitt in den Sicherheitsgurt eindringt.

Auch das Erzeugen der Öffnung kann unter Erwärmung des Teilabschnitts des Sicherheitsgurtes, in dem die Öffnung entstehen soll, erfolgen.

Die Erfindung wird im Folgenden anhand von Ausführungsbeispielen unter Bezugnahme auf die Figuren näher erläutert. Es zeigen:
- Figur 1: eine in einem Fahrzeug installierte beispielhafte Sicherheitsgurtanordnung;
- Figur 2: einen der Sensorbereiche der Sicherheitsgurtanordnung aus Figur 1;
- Figur 3: eine perspektivische Schnittdarstellung entlang der Linie A-A des Sensorbereiches der Figur 2;
- Figur 4: eine weitere Schnittdarstellung des Sensorbereiches der Figur 2;
- Figur 5: den Sensorbereich der Figur 4 mit einem Vergussmaterial;
- Figur 6: einen Schritt bei einer alternativen Herstellung der Sicherheitsgurtanordnung;
- Figur 7: eine Vorrichtung zur Herstellung der Sicherheitsgurtanordnung;
- Figur 8: eine perspektivische Schnittdarstellung eines Sensorbereiches eines Sicherheitsgurtes einer Sicherheitsgurtanordnung gemäß der Erfindung ;
- Figur 9: eine Abwandlung der Figur 8; und
- Figur 10: eine Anordnung zur Herstellung einer Öffnung in einem Sicherheitsgurt zum Anordnen eines Sensors.

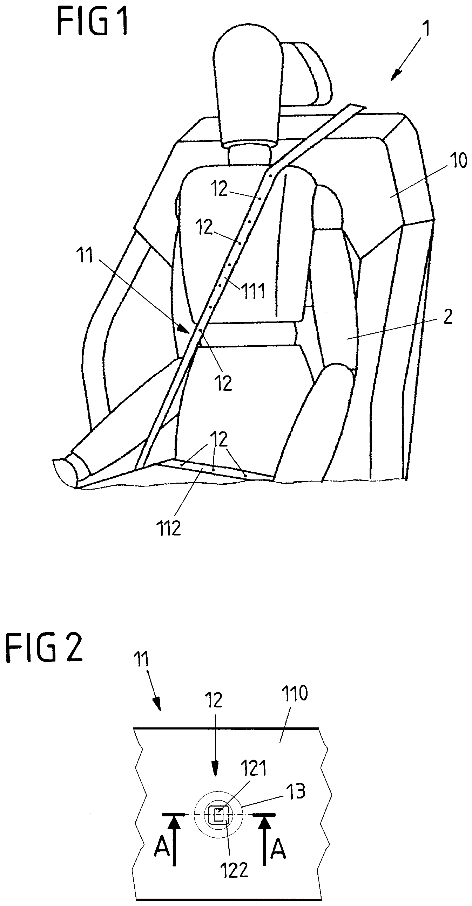

Figur 1 stellt eine in einem Kraftfahrzeug installierte und einem Fahrzeugsitz 10 zugeordnete erfindungsgemäße Sicherheitsgurtanordnung 1 dar. Die Sicherheitsgurtanordnung 1 umfasst einen Sicherheitsgurt 11, der einen Oberkörperabschnitt 111 und einen Beckenabschnitt 112 aufweist. Der Oberkörperabschnitt 111 verläuft in an sich bekannter Weise zwischen einem oberen Gurtumlenkelement (nicht dargestellt), das zum Beispiel in eine Fahrzeugseitenstruktur (etwa die B-Säule des Fahrzeugs) integriert ist, und einem unteren Gurtumlenkelement (ebenfalls nicht dargestellt), das insbesondere benachbart zu einer Sitzfläche des Fahrzeugsitzes 10 angeordnet und z.B. mit einer Gurtzunge kombiniert ist. Der Beckenabschnitt 112 verläuft zwischen dem unteren Gurtumlenkelement und einer Gurtverankerung, die zum Beispiel mit einer Fahrzeugseitenstruktur verbunden ist. Mögliche grundsätzliche Ausgestaltungen der Sicherheitsgurtanordnung 1 sind in der oben bereits erwähnten deutschen Patentanmeldung 10 2014 211 501 vom 16.06.2014 beschrieben.

Der Sicherheitsgurt 11 umfasst des Weiteren mehrere Sensoren 12, die sich gemäß Figur 1 sowohl an dem Oberkörperabschnitt 111 des Sicherheitsgurtes 11 als auch an dem Beckenabschnitt 112 befinden können. Denkbar ist allerdings auch, dass der Sicherheitsgurt 11 nur in seinem Oberkörperabschnitt 111 Sensoren 12 aufweist und nicht auch in seinem Beckenabschnitt 112. Grundsätzlich möglich ist natürlich auch der umgekehrte Fall, wonach nur der Beckenabschnitt 112 Sensoren 12 aufweist. Anstelle einzelner Sensoren können jeweils natürlich wie oben bereits erläutert Sensoreinheiten (Sensorknoten) mit jeweils mehreren Sensoren und z.B. auch weiteren elektronischen Komponenten vorgesehen sein. Die Sensoren 12 dienen insbesondere zur Überwachung von Vitalfunktionen des Gurtbenutzers 2. Denkbar ist auch, dass zumindest einige der Sensoren 12 in Form von akustischen Wandlern ausgebildet sind, die z.B. ebenfalls zur Überwachung von Vitalfunktionen und/oder als Mikrofone zur Erfassung von Sprachbefehlen verwendet werden oder als Teil einer Freisprecheinrichtung vorgesehen sind. Möglich ist auch, dass es sich zumindest bei einigen der Sensoren 12 um unterschiedliche Sensortypen handelt.

Die Sensoren 12 sind jeweils in einer Einbuchtung 13 (Vertiefung) einer Innenseite 110 des Sicherheitsgurtes 11 angeordnet, wobei die Innenseite 110 nach dem Anlegen dem Fahrzeuginsassen 2 zugewandt ist und zumindest abschnittsweise an diesem anliegt.

Einer der Sensoren 12 der Figur 1 ist im Detail in Figur 2 dargestellt, die eine Draufsicht auf die Innenseite 110 (die Körperseite) des Sicherheitsgurtes 11 zeigt. Der dort gezeigte beispielhafte Sensor 12 weist ein Sensorelement 121 auf, das auf einer Leiterplatte 122 angeordnet ist. Der gesamte Sensor 12 befindet sich in der Einbuchtung 13, wobei die Tiefe der Einbuchtung 13 so bemessen ist, dass der Sensor 12 nicht aus der Einbuchtung 13 herausragt, d.h. der Sensor 12 ragt nicht über eine Fläche hinaus, die durch einen die Einbuchtung 13 umgebenden Abschnitt der Innenseite 110 des Sicherheitsgurtes 11 gebildet ist.

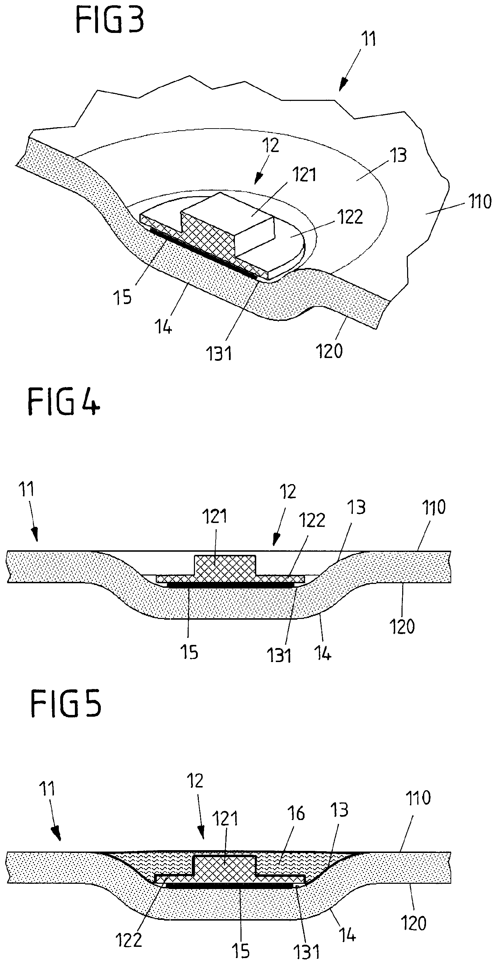

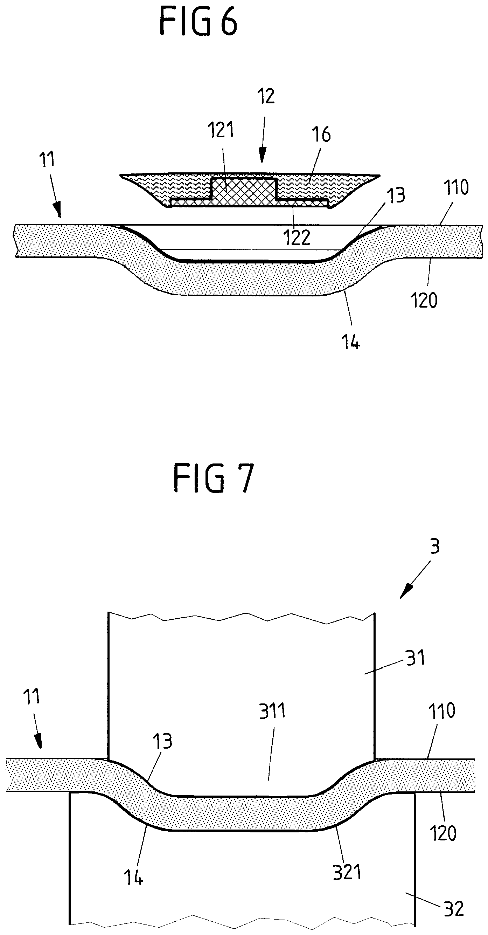

Dies ist insbesondere auch den Schnittdarstellungen der Figuren 3 und 4 zu entnehmen, die jeweils einen Schnitt entlang der Linie A-A in Figur 2 zeigen (Figur 3 in perspektivischer Darstellung). Die Einbuchtung 13 ist vorliegend mit einer zumindest teilweise kreisförmigen Kontur ausgebildet, deren Durchmesser von oben bis zu einem Bodenbereich 131 der Einbuchtung 13 abnimmt. Entsprechend besitzt die Einbuchtung 13 eine schüssel- oder tellerartige Form. Denkbar sind selbstverständlich auch andere Formen der Einbuchtung 13, z.B. kann die Einbuchtung 13 auch hohlzylindrisch ausgebildet sein. Die Einbuchtung 13 entsteht nicht durch Materialabtrag des Sicherheitsgurtes 11, sondern durch Eindrücken eines Bereiches von der Innenseite 110 her; vgl. Figur 7. Entsprechend zeigt der Sicherheitsgurt 11 im Bereich der Einbuchtungen 13 jeweils korrespondierende Ausbuchtungen 14 seiner Außenseite 120.

Die Sensoren 12 sind jeweils in ihrer Einbuchtung 13 befestigt, wobei die Befestigung insbesondere jeweils stoffschlüssig zumindest mit dem Bodenbereich 131 der Einbuchtung 13 erfolgt. Beispielsweise wird ein Klebstoff 15 verwendet.

Nach dem Anordnen des Sensors 12 in der Einbuchtung 13 wird gemäß Figur 5 ein Schutzmaterial in Form eines Vergussmaterials 16 in die Einbuchtung 13 eingefüllt, um den Sensor 12 vor äußeren Einwirkungen zu schützen und/oder die Form der Einbuchtung 13 zu stabilisieren. Das Vergussmaterial 16 füllt insbesondere den von dem Sensor 12 nicht eingenommenen Bereich der Einbuchtung 13 zumindest im Wesentlichen vollständig aus. Darüber hinaus schließt das Vergussmaterial 16 mit dem die Einbuchtung 13 umgebenden Abschnitt des Sicherheitsgurtes 11 zumindest im Wesentlichen ab, so dass die Innenseite 110 des Sicherheitsgurtes 11 eine zumindest näherungsweise glatte Struktur erhält.

Alternativ kann der Sensor 12 vor seinem Einsetzen in die Einbuchtung 13 des Sicherheitsgurtes 11 mit dem Vergussmaterial 16 (als Umkapselung) versehen werden, so dass eine Einheit aus dem Sensor 12 und dem Vergussmaterial 16 gebildet wird; vgl. Figur 6. Diese Einheit wird dann in die Einbuchtung 13 eingesetzt und dort befestigt (wie erwähnt insbesondere stoffschlüssig). Das Vergussmaterial 16 kann in diesem Fall eine Form (eine äußere Kontur) besitzen, die derjenigen der Einbuchtung 13 entspricht, so dass die Einbuchtung 13 nach dem Einsetzen der Einheit aus dem Sensor 12 und dem Vergussmaterial 16 analog zu Figur 5 zumindest näherungsweise vollständig ausgefüllt ist. Das Vergießen des Sensors 12 mit dem Vergussmaterial 16 erfolgt insbesondere unter Verwendung einer entsprechend der Einbuchtung 13 ausgebildeten Form.

Figur 7 illustriert eine Möglichkeit zur Erzeugung der Einbuchtungen 13 für die Sensoren 12. Danach wird ein Werkzeug 3 verwendet, das ein oberes Werkzeugteil in Form eines Stempels 31 sowie ein unteres Werkzeugteil in Form einer Matrize 32 umfasst, wobei der Stempel 31 und die Matrize 32 insbesondere aus einem Metall oder einem Kunststoff ausgebildet sind. Die Matrize 32 besitzt eine Vertiefung 321, die komplementär zu der mit der gewünschten Einbuchtung 13 korrespondierenden Ausbuchtung 14 des Sicherheitsgurtes 11 ausgeformt ist. Der Stempel 31 wiederum besitzt einen Endabschnitt 311, der komplementär zur gewünschten Einbuchtung 13 geformt ist.

Zur Herstellung der Einbuchtung 13 wird der Sicherheitsgurt 11 zwischen dem Stempel 31 und der Matrize 32 positioniert und eine Relativbewegung des Stempels 31 und der Matrize 32 aufeinander zu erzeugt, um einen Abschnitt des Sicherheitsgurtes 11 in die Vertiefung 321 der Matrize 32 zu drücken (nach Art des Tiefziehens). Beispielsweise wird der Stempel 31 auf die feststehende Matrize 32 zu bewegt, so dass der Endabschnitt 311 des Stempels 31 einen Teilbereich des Sicherheitsgurtes 11 in die Vertiefung 321 der Matrize 32 drückt und somit die gewünschte Einbuchtung 13 erzeugt.

Die Herstellung der Einbuchtung 13 erfolgt insbesondere unter Temperaturerhöhung zumindest desjenigen Abschnitts des Sicherheitsgurtes 11, in dem die Einbuchtung 13 entstehen soll. Die Erwärmung des Sicherheitsgurtes 11 kann die Herstellung der Einbuchtung 13 erleichtern und/oder einer Beschädigung des Gewebes des Sicherheitsgurtes 11 bei der Herstellung der Einbuchtung 13 entgegenwirken. Beispielsweise werden der Stempel 31 und/oder die Matrize 32 zumindest teilweise erwärmt, um für eine Erwärmung des Sicherheitsgurtes im Bereich der vorgesehenen Einbuchtung 13 zu sorgen. Alternativ oder zusätzlich kann der Sicherheitsgurt 11 zumindest in dem Bereich, in dem die Einbuchtung 13 entstehen soll, vorgewärmt in dem Werkzeug 3 angeordnet werden.

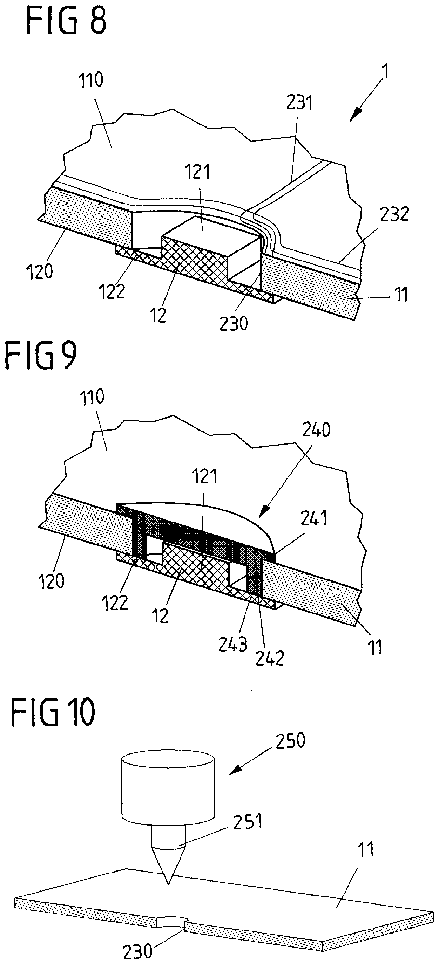

Figur 8 bezieht sich auf eine Sicherheitsgurtanordnung gemäß der Erfindung, wobei analog zur Figur 3 ein Sensorbereich des Sicherheitsgurtes 11 der Sicherheitsgurtanordnung 1 dargestellt ist. Der Sensor 12 ist nicht in einer Einbuchtung, sondern in einer in dem Sicherheitsgurt 11 ausgebildeten Durchgangsöffnung 230 angeordnet. Die Öffnung 230 wird durch Verdrängen (Auseinanderdrücken) von Kett- und Schussfäden 231, 232 eines Gewebematerials, aus dem der Sicherheitsgurt 11 hergestellt ist, erzeugt. Denkbar ist, dass der Sensor 12 nicht das gesamte auf diese Weise erzeugte Volumen der Öffnung 230 einnimmt, wobei das nicht vom Sensor 12 ausgefüllte Volumen zumindest teilweise mit einem Schutzmaterial (z.B. einem Kunststoff oder einem Kleber) befüllt sein kann.

Des Weiteren ist denkbar, dass die Kett- und Schussfäden 231, 232 zumindest eine gewisse Elastizität aufweisen, so dass sie sich nach Erzeugen der Öffnung 230 und Einsetzen des Sensors 12 in die Öffnung 230 in ihrer ursprüngliche Lage zurückbewegen und somit einen Teil der ursprünglichen Öffnung wieder verschließen und zumindest teilweise auch zur Anlage an dem Sensor 12 kommen.

Der Sensor 12 ist z.B. wie oben beschrieben an dem Sicherheitsgurt 11 festgelegt, z.B. stoff- oder formschlüssig. Gemäß Figur 8 befindet sich ein Abschnitt einer Leiterplatte 122 des Sensors 12 außerhalb der Öffnung 230, wobei dieser Abschnitt der Leiterplatte 122 an der Außenseite 120 des Sicherheitsgurtes 11 anliegt und z.B. dort festgelegt ist. Der Sensor 12 ragt somit von der Außenseite 120 in die Öffnung 230 hinein, so dass seine Sensierrichtung nach Anlegen des Sicherheitsgurtes 11 auf den Fahrzeuginsassen weist. Insbesondere befindet sich das Sensorelement 121 des Sensors 12 zumindest näherungsweise vollständig in der Öffnung 230. Die Leiterplatte 122 kann auch Bestandteil eines Gehäuses sein, in dem der Sensor 12 angeordnet ist. Beispielsweise bildet die Leiterplatte 122 einen Boden des Gehäuses aus.

Figur 9 zeigt eine Abwandlung der Figur 8. Danach ist zur Befestigung des Sensors 12 in der Öffnung 230 ein (nach Art eines Deckels ausgebildetes) Haltelement 240 vorgesehen, das einen ersten, flanschartigen Abschnitt 241 aufweist, der an der Innenseite 110 des Sicherheitsgurtes 11 anliegt. Darüber hinaus umfasst das Haltelement 240 einen zweiten Abschnitt 242, mit dem es von der Innenseite 110 her in die Öffnung 230 hineinragt. Dieser zweite Abschnitt 242 ist mit der Leiterplatte 122 des Sensors 12 verbunden, insbesondere über eine Stirnseite 243 des zweiten Abschnitts 242, die auf der Leiterplatte 122 aufsetzt. Somit ist der Sensor 12 nach Art eines Niets an dem Sicherheitsgurt 11 festgelegt. Die Verbindung des Haltelement 240 mit dem Sensor 12 erfolgt z.B. stoffschlüssig (etwa per Verkleben oder Ultraschallschweißen) oder per Rast- oder Crimpverbindung.

Fig. 10 illustriert die Herstellung der Öffnung 230 in dem Sicherheitsgurt 11. Danach wird ein Werkzeug in Form eines Dorns 250 verwendet, wobei der Dorn 250 einen teilweise konischen Abschnitt in Form einer Spitze 251 aufweist. Der Dorn 250 wird nun so bewegt (insbesondere senkrecht zu zumindest einem Abschnitt des Sicherheitsgurtes 11), dass seine Spitze 251 in das Gewebematerial des Sicherheitsgurtes 11 eindringt. Die Spitze 251 ist so beschaffen, dass sie beim Eindringen in das Gewebematerial dessen Kett- und Schussfäden lediglich auseinanderdrückt und nicht durchtrennt. Dies ermöglicht eine Herstellung von Sensoröffnungen in dem Sicherheitsgurt 11 ohne Beeinträchtigung (zumindest ohne wesentliche Beeinträchtigung) seiner Zugfestigkeit.

## Patentansprüche

1. Sicherheitsgurtanordnung für ein Kraftfahrzeug, mit
- einem Sicherheitsgurt (11);
- mindestens einem Sensor (12),
**dadurch gekennzeichnet, dass**
der Sensor (12) zumindest teilweise in einer zumindest im Wesentlichen durch Verdrängung von Material des Sicherheitsgurtes (11) gebildeten Öffnung (230) des Sicherheitsgurtes (11) angeordnet ist, wobei der Sicherheitsgurt (11) aus einem gewebten Material besteht und die Öffnung (230) durch Auseinanderdrücken von Materialfäden (231, 232) des gewebten Materials gebildet ist.

2. Sicherheitsgurtanordnung nach Anspruch 1, **dadurch gekennzeichnet, dass** es sich bei der Öffnung (230) um eine Durchgangsöffnung handelt.

3. Sicherheitsgurtanordnung nach Anspruch 1 oder 2, **dadurch gekennzeichnet, dass** Materialfäden (231, 232) des gewebten Materials an dem Sensor (12) anliegen.

4. Sicherheitsgurtanordnung nach einem der vorhergehenden Ansprüche, **dadurch gekennzeichnet, dass** ein vom Sensor (12) nicht eingenommenes Volumen der Öffnung (230) zumindest teilweise mit einem Schutzmaterial (16) versehen ist.

5. Sicherheitsgurtanordnung nach einem der vorhergehenden Ansprüche, **gekennzeichnet durch** ein Halteelement (240), das mit einem ersten Abschnitt (241) an einer Seite des Sicherheitsgurtes (11) anliegt und mit einem zweiten Abschnitt (242) in die Öffnung (230) hineinragt und mit dem Sensor (12) verbunden ist.

6. Verfahren zur Herstellung einer Sicherheitsgurtanordnung nach einem der vorhergehenden Ansprüche, mit den Schritten:
- Bereitstellen eines Sicherheitsgurtes (11);
- Verdrängen von Material des Sicherheitsgurtes (11) derart, dass eine Öffnung (230) in dem Sicherheitsgurt (11) entsteht, wobei der Sicherheitsgurt (11) aus einem gewebten Material besteht und das Verdrängen von Material des Sicherheitsgurtes (11) durch Auseinanderdrücken von Materialfäden (231, 232) des gewebten Materials erfolgt; und
- Anordnen eines Sensors (12) in der Öffnung.

7. Verfahren nach Anspruch 6, **dadurch gekennzeichnet, dass** das Auseinanderdrücken der Materialfäden (231, 232) mit Hilfe eines Werkzeuges (250) erfolgt, wobei das Werkzeug (250) einen konischen Abschnitt (251) aufweist und so bewegt wird, dass der konische Abschnitt (251) in den Sicherheitsgurt (11) eindringt.

## Claims

1. Seatbelt arrangement for a motor vehicle, having
- a seatbelt (11);
- at least one sensor (12),
**characterized in that**
the sensor (12) is at least partially arranged in an opening (230) of the seatbelt (11), said opening being at least substantially formed by displacement of material of the seatbelt (11), wherein the seatbelt (11) is composed of a woven material and the opening (230) is formed by pushing apart material threads (231, 232) of the woven material.

2. Seatbelt arrangement according to Claim 1, **characterized in that** the opening (230) is a passage opening.

3. Seatbelt arrangement according to Claim 1 or 2, **characterized in that** material threads (231, 232) of the woven material lie against the sensor (12).

4. Seatbelt arrangement according to one of the preceding claims, **characterized in that** a volume of the opening (230) that is not taken up by the sensor (12) is at least partially provided with a protective material (16).

5. Seatbelt arrangement according to one of the preceding claims, **characterized by** a holding element (240) which lies with a first portion (241) against one side of the seatbelt (11) and projects as a second portion (242) into the opening (230) and is connected to the sensor (12).

6. Method for producing a seatbelt arrangement according to one of the preceding claims, with the following steps:
- providing a seatbelt (11);
- displacing material of the seatbelt (11) in such a manner that an opening (230) arises in the seatbelt (11), wherein the seatbelt (11) is composed of a woven material and the material of the seatbelt (11) is displaced by pushing apart material threads (231, 232) of the woven material; and
- arranging a sensor (12) in the opening.

7. Method according to Claim 6, **characterized in that** the material threads (231, 232) are pushed apart with the aid of a tool (250), wherein the tool (250) has a conical portion (251) and is moved in such a manner that the conical portion (251) penetrates the seatbelt (11).

## Revendications

1. Ensemble formant ceinture de sécurité, destiné à un véhicule automobile, comprenant
- une ceinture de sécurité (11) ;
- au moins un capteur (12),
**caractérisé en ce que**
le capteur (12) est disposé au moins partiellement dans une ouverture (230) de la ceinture de sécurité (11) qui est formée au moins sensiblement par déplacement de matière de la ceinture de sécurité (11), la ceinture de sécurité (11) comprenant un matériau tissé et l'ouverture (230) étant formée par écartement de fils (231, 232) du matériau tissé.

2. Ensemble formant ceinture de sécurité selon la revendication 1, **caractérisé en ce que** l'ouverture (230) est une ouverture de passage.

3. Ensemble formant ceinture de sécurité selon la revendication 1 ou 2, **caractérisé en ce que** les fils (231, 232) du matériau tissé viennent en appui sur le capteur (12).

4. Ensemble formant ceinture de sécurité selon l'une des revendications précédentes, **caractérisé en ce qu'**un volume de l'ouverture (230) qui n'est pas occupé par le capteur (12) est au moins partiellement pourvu d'un matériau de protection (16).

5. Ensemble formant ceinture de sécurité selon l'une des revendications précédentes, **caractérisé par** un élément de retenue (240) qui vient en appui, par une première partie (241), sur un côté de la ceinture de sécurité (11) et qui fait saillie, par une deuxième partie (242), dans l'ouverture (230) et est relié au capteur (12).

6. Procédé de fabrication d'un ensemble formant ceinture de sécurité selon l'une des revendications précédentes, comprenant les étapes suivantes :
- produire une ceinture de sécurité (11) ;
- déplacer le matériau de la ceinture de sécurité (11) de manière à former une ouverture (230) dans la ceinture de sécurité (11), la ceinture de sécurité (11) comprenant un matériau tissé et le déplacement de matériau de la ceinture de sécurité (11) étant effectué par écartement des fils (231, 232) du matériau tissé ; et
- disposer un capteur (12) dans l'ouverture.

7. Procédé selon la revendication 6, **caractérisé en ce que** l'écartement des fils de matériau (231, 232) est effectué au moyen d'un outil (250), l'outil (250) comportant une partie conique (251) et étant déplacé de manière à ce que la partie conique (251) pénètre dans la ceinture de sécurité (11).
